# EUROPEAN PATENT SPECIFICATION

(11) **EP 3 846 887 B1**
(45) Date of publication and mention of the grant of the patent: **21.02.2024**
(21) Application number: 19858179.5
(22) Date of filing: 05.09.2019
(51) Int. Cl.: A61M 16/00, G01F 1/44, A62B 7/00

(54) **NEGATIVE AIR PRESSURE DEVICES AND USES THEREOF**
UNTERDRUCKVORRICHTUNGEN UND DEREN VERWENDUNGEN
DISPOSITIFS À PRESSION D'AIR NÉGATIVE ET LEURS UTILISATIONS

(30) Priority: 05.09.2018 US 201862727331 P
(43) Date of publication of application: 14.07.2021
(73) Proprietor: Vikare, LLC, Waunakee, Wisconsin 53597 (US)
(72) Inventor: DOS SANTOS, Icaro, Waunakee, Wisconsin 53597 (US)
(74) Representative: Algemeen Octrooi- en Merkenbureau B.V.
(86) International application number: PCT/US2019/049704
(87) International publication number: WO 2020/051304

(56) References cited:
- WO-A1-00/13731
- WO-A1-93/23102
- WO-A1-2014/005191
- FR-A1- 2 988 005
- US-A- 3 653 379
- US-A1- 2005 028 811
- US-A1- 2009 228 018
- US-A1- 2011 319 783
- US-A1- 2013 199 535
- US-A1- 2013 199 535

## Description

### FIELD OF THE DISCLOSURE

Provided herein are negative air pressure devices and uses thereof. In particular, provided herein are negative air pressure devices that modulate CO₂ delivery for use in the treatment of sleep apnea and related conditions.

### BACKGROUND

Sleep apnea is a serious public health problem. The prevalence of this disease is approximately 1.0 billion people worldwide. Continuous Positive Airway Pressure (CPAP) equipment is still considered the standard treatment for obstructive sleep apnea (OSA) and even some types of central and mixed sleep apnea, but positive airway pressure is not tolerated by many patients and therefore, CPAP is underutilized. In other words, CPAP is established as a highly efficacious treatment for OSA. However, its effectiveness has been limited by poor adherence. In fact, many patients with sleep apnea consider CPAP positive pressure to be extremely uncomfortable and drop out in less than 6 months. Between 46 and 83% of patients with obstructive sleep apnea were reported as non-adherent to treatment. Patients often experience nasal discomfort, air dryness, excess humidity, problems with mask fit, mask leak, high pressure discomfort, congestion, noise and claustrophobia.

International patent application WO93/23102 A1 discloses a non-wasting respiratory stimulator for providing a CO2 enriched fresh air mixture. The respirator comprises a breathing port, a vent port and a mixing chamber, and methods of treating hypocapnia of various etiology, comprising administering a mixture of exhaled CO2 and fresh air, utilizing the non-wasting respiratory stimulator.

United States patent application US 2005/028811 A1 discloses multitask medical treatment respiratory apparatus having a mask defining a chamber wherein the mask has a first port, a second port and a venting valve. A reservoir bag having a first opening is in communication with the first port or there may be a venturi device in communication with a second opening of the reservoir bag. One or more gas sources are in communication with the venturi device.

United States patent application US 3653379 A discloses a breathing apparatus in which gas is delivered to a patient under pressure intermittently. Air or oxygen delivered into a venturi tube includes an adjustable nozzle for changing pressure as required for a particular patient's demand.

What is needed are devices for treating sleep apnea that are simple, lightweight, and well tolerated by users.

### SUMMARY

Provided herein are negative air pressure devices and uses thereof. In particular, provided herein are negative air pressure devices that modulate CO₂ delivery for use in the treatment of sleep apnea and related conditions.

Provided herein is an improved treatment for sleeping apnea that is much more comfortable and effective than existing devices such as CPAP. In some embodiments, provided herein are intermittent negative air pressure or continuous negative air pressure devices that utilize the Bernoulli principle and Venturi effect to deliver CO₂ enriched air to subjects in need of treatment for sleep apnea. In some embodiments, the devices described herein do not require cleaning, are comfortable, and do not require electricity to operate.

Accordingly to a first aspect, the current invention provides a negative air pressure device according to claim 1.

In some embodiments, the device further comprises a face mask in operable communication with the transport tube (e.g., a face mask comprising valves). In some embodiments, the transport tube further comprises an adjustment component configured to move the transport tube in closer or further proximity to the mixing chamber (e.g., an infinity screw or a motor).

In some embodiments, the inlet further comprises a valve configured to control the cross-sectional area of the inlet and/or flow of gas into the inlet. The present disclosure is not limited to particular valves. Examples include, but are not limited to, a two-way valve (e.g., a butterfly valve) or a one-way valve (e.g., an umbrella valve). In some embodiments, the device comprises two or more (e.g., 2 3, 4, 5, or more) inlets and/or outlets. In some embodiments, the valve is configured to move with the axis of the inlet (e.g., via a screw motor). In some embodiments, the outlet is covered by a filter. In some embodiments, a secondary flow inlet causes the flow to enter tangentially with respect to the mixing chamber. In some embodiments, the secondary flow enters radially with respect to the main flow. In some embodiments, the outlet/inlet comprises a valve. The present disclosure is not limited to a location of the inlet. In some embodiments, the inlet is located on the top of the mixing chamber, on the side of the transport tube, or another location. In some embodiments, the inlet comprises an actuator that controls movement of air through the actuator.

The constriction generates negative pressure via the venturi effect.

In some embodiments, the device is enclosed in a case. In some embodiments, the device further comprises an external source of gas (e.g., CO₂ or air) in operable communication with the device. In some embodiments, the device provides intermittent, periodic, or continuous negative air pressure (e.g., via the venturi effect). In some embodiments, the device traps CO₂ in the mixing chamber.

Further embodiments provide a system according to claim 12.

In some embodiments, the processor controls CO₂ levels by controlling flow of atmospheric air or enriched CO₂ air into the device through the inlet.

Yet other unclaimed embodiments provide a method of treating sleep apnea in a subject, comprising: applying the device or system described herein to a subject in need thereof. The devices, systems, and methods described herein are suitable for use in treating any type of sleep apnea (e.g., including but not limited to, obstructive, central or mixed sleep apnea).

Additional embodiments are described herein.

### DESCRIPTION OF THE FIGURES

FIG. 1 shows an exemplary device of embodiments of the present invention.
FIG. 2 shows an exemplary device of embodiments that are not part of the present invention with a side inlet.
FIG 3 shows an exemplary device of embodiments of the present invention with an inlet valve.
FIG. 4 shows an exemplary device of embodiments that are not part of the present invention with an inlet valve.
FIG. 5 shows an exemplary device of embodiments that are not part of the present invention with a Y shape venturi effect component.
FIG. 6 shows an exemplary device of embodiments of the present invention with a symmetrical venturi effect component
FIG. 7 shows an exemplary device of embodiments of the present invention with a symmetrical venturi effect component located at an inlet.
FIG. 8 shows an exemplary device of embodiments of the present invention with a symmetrical shape venturi effect component located in a valve in a secondary inlet.
FIG. 9 shows an exemplary device of embodiments of the present invention with a blower component.
FIG. 10 shows an exemplary device of embodiments of the present invention with a tangential secondary flow.
FIG. 11 shows an exemplary mask for use with devices of embodiments of the present invention.
FIG. 12 shows an algorithm logic flow for controlling CO₂ levels.
FIG 13 shows an exemplary CAD embodiment of the main body with a constraint in the middle to improve the mixing of the gases.
FIG 14 shows a picture of the device constructed according to the CAD design shown in FIG 13.
FIG 15 shows experimental data obtained using the device of FIG 14.

### DETAILED DESCRIPTION

Provided herein are negative air pressure devices and uses thereof. In particular, provided herein are negative air pressure devices that modulate CO₂ delivery for use in the treatment of sleep apnea.

The devices described herein are based, in part, on the Bernoulli principle, Venturi effect, and mass and energy transfer equations. Common realizations of these concepts in other systems are (a) ejectors, (b) injectors, (c) air jets, (d) eductors, (e) jet pumps, (f) carburetors, (g) cyclonic separators etc. The Bernoulli theorem teaches us that the energy of flowing fluid (for small height differences) remains constant, but this energy can take a form of either kinetic energy or potential energy (in the pure form of pressure for horizonal flow). In order words, if a fluid accelerates its pressure will drop and vice-versa, while the total energy remains unchanged. For air flowing in a tube, the changes in pressure and velocity can be made to occur by narrowing or widening the tube or sections of the tube. For example, one can decrease the pressure and thus increase the velocity of a fluid by creating a reduction in the cross-section area (a throat). The cross-section area of the tube or of the constraint can have any shape, e.g. circular or rectangular. The same effect of changing pressure and kinetic energy can be created using nozzles. Because of the increase in the velocity, the pressure of the moving fluid becomes lower than of any surrounding fluids, which cause the latter to flow toward the area of lower pressure similarly the way air flows into vacuum.

The Bernoulli principle and the Venturi effect show that regions where a fluid has a higher velocity cause a negative pressure. In other words, an increase in the speed of a fluid occurs simultaneously with a decrease in pressure or a decrease in the fluid's potential energy. In the present disclosure, these principals are used to create a region of low pressure that can be used to control the mixing between atmospheric air and either exhaled air and/or other gases such as CO₂ or O₂. Because the devices described herein allow for control of the lower pressure, one can control both the intensity and the direction of the secondary gas and thus the mixing of the gas. Thus, contamination of the non-disposable parts is avoided. Moreover, there is no need to use valves to control the amount of CO₂ inside the mixing chamber, however one can still use them if desired. Also, in some embodiments, the tube throat is constrained by an exterior actuator without putting it in direct contact with the exhaled air. In some embodiments, a valve, diaphragm, needle of any other means is used to reduce the cross-section in the tube both/either at the exit/exits and/or in the inlets (primary or secondary). More or less cross-section area provides a more or less differential pressure and hence more or less mixing. Again, this avoids contamination since the air only travels from the outside (atmospheric) to the inside of the mixing chambers if there is a constraint in the tube.

The devices described herein do not need to be rigid and can be constructed of any type of appropriate fiber or other material, e.g., paper, cardboard, plastic, metal or alloy (e.g., stainless steel), and ceramic. Because the devices can be constructed of an absorbing material, the device can control excess moisture (a common complain of CPAP users) and be disposable so cleaning is not needed (another common complain of CPAP users) and inexpensive to manufacture. Because the pressures involved are small (typically less than 20 mmHg), the mask and/or nasal pillows do not need to be very tight to avoid leaks, which are a source of complaint among users of PAP machines.

In some embodiments of the device, the venturis are asymmetrical because in most of the applications a fluid can be adequately mixed in only one direction. However, in the devices described herein, they can be symmetrical, so the atmospheric air is mixed inside of the device in both the inhale and exhale phases in a similar fashion. Another advantage of the devices of the present disclosure is that they do not require a fan or blower to create the negative pressure but rather uses the kinetic energy of the inspired and/or expired air from the user to create the differential pressure. Thus, no third source of energy is necessary.

Further provided herein is a computer-controlled algorithm to control the amount of CO₂ based on one or more parameters (e.g., concentration of CO₂ in the device or mask, detection of apneic event, etc.). The apneic event can be detected by changes or lack of variation of flow, pressure, gas concentration, temperature, humidity, electrical resistivity, sound and/or electromagnetic changes inside or outside of the chamber and/or the mask. Alternatively, or in combination, it can be detected by pulse oximetry, chest movement strain gage attached to the device or patient, chest movement image, and/or video. However, it is preferable not to be in contact with the interior of the primary or secondary chambers to avoid contamination and hence the need of cleaning.

In some embodiments, the devices described herein treat sleep apnea by delivering amounts of CO₂ either generated by patients during the exhale phase of the respiration or by an external gas reservoir or by concentrating the natural occurred CO₂ in the atmosphere. In some embodiments, the amount of CO₂ delivered to the patient is controlled based on the measurements of the individuals' respiratory instability, e.g., hypoapnea or apnea events.

Accordingly, provided herein are Variable Negative Air Pressure device (VNAP) or Constantly Negative Air Pressure (CNAP) devices for use in treating sleep apnea. While the present devices are exemplified with controlling the amount of CO₂ delivered to the patients during the respiratory cycle in order to stabilize their respiration, the present disclosure is not limited to CO₂ regulation. In some embodiments, the device is used to control the amount of any other gases exhaled by the patient such as, for example, O₂, N₂, etc. Exemplary devices are described herein.

In some embodiments, the VNAP or CNAP comprises at least one of(e.g., all three) modules: (1) a chamber comprise a venturi, air injector, air ejector or educator based on the Bernoulli principle to vary the mixing between the fresh/atmosperic air and CO₂ by using one or more actuators; (2) one or more sensors to detect apnea or hypoapnea; and (3) a control mechanism for delivering the required amount of CO₂ used to stabilize the patients' respiration.

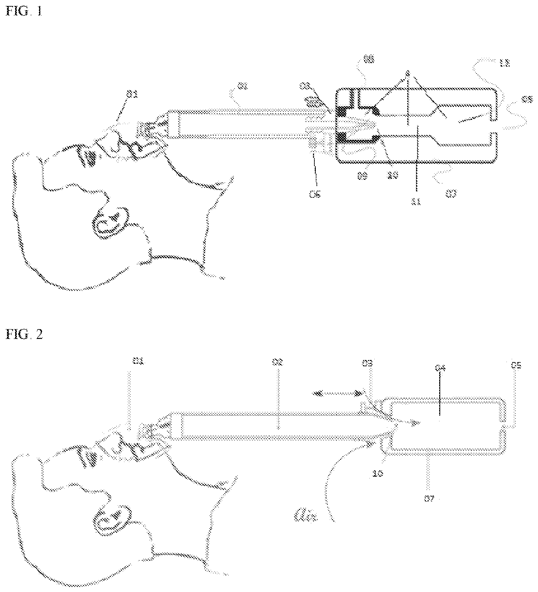

Figure 1 shows a schematic drawing of one of the embodiments that used the air ejector principle. In this case, the amount of fresh air is controlled by the inserted tube position with respect to the primary mixing chamber.

FIG. 1 shows mask 1, transport tube 2, transport tube adjustment component 3, mixing chamber 4, outlet/inlet 5, gas inlet 8, nozzle 10, actuator 6, and case 7.

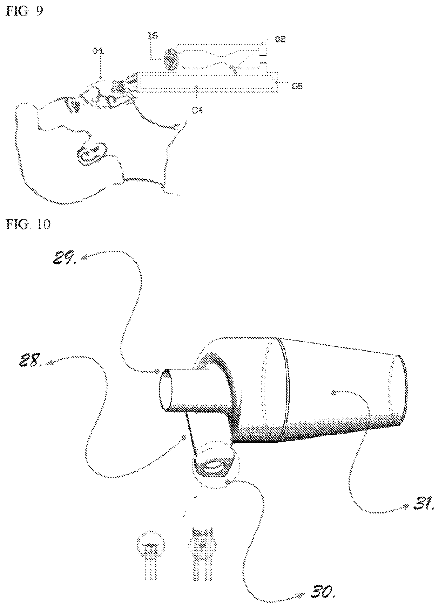

Still referring to FIG. 1, the present disclosure is not limited to particular designs for mask 1. The mask can be any of the commonly used PAP masks or nose pillow or any other mask suitable to be used in exchanging gases from and to the subject. However, since the involved pressures are not as high as occurred in the PAP machines, it is not necessary to be as tightly attached to the users' face or nostrils as in the case of the PAP devices and thus it is more comfortable and more flexible. In some embodiments, mask 1 comprises a plurality of valves as shown in FIG. 10. In some embodiments, the mask is obtained from commercial sources (e.g., from 3M corporation, St. Paul, MN, Honeywell, Morris Plains, NJ or other sources).

Still referring to FIG. 1, in some embodiments, the device comprises a transport tube adjustment component configured to move the nozzle in closer proximity to the mixing chamber. This allows for control of negative pressure and the corresponding flow rate. The present disclosure is not limited to particular adjustment components. Examples include, but are not limited to, an electric motor with or without gears or chains, an infinity screw (e.g., 370C-08700-N-CV, Transmotec, Boston, MA) or a piezoelectric motor (e.g., Piezo LEGS^{®} Linear 6N, PiezoMotor, Uppsala, Sweden), pneumatic actuator (e.g., MA-250 X 0.25-DA-RS, Universal Power Conversion, Savage, MN), or any other material that can contract, expand, shift or rotate due to stimulus such as voltage, electrical current, and temperature (e.g., artificial muscle).

Still referring to FIG. 1, the present disclosure is not limited to particular materials for transport tube 2. The tube can be made of any biocompatible material or medical grade material, rigid or flexible.

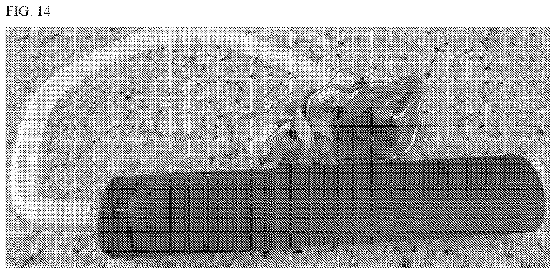

Still referring to FIG. 1, a gas inlet 8 is shown. The gas inlet provides the atmospheric air or any other gas or combination of gases from a third source such as, for example, CO₂, O₂, N₂, etc. The inlet does not need to have a valve. However, in some embodiments, a valve is added (not shown in FIG. 1) to control its cross-section area and hence the flow rate. The present disclosure is not limited to particular valves. In some embodiments, the valve is a two-way valve, such as, for example, a butterfly valve or one-way valve such as, for example, an umbrella valve that allows only the flow to inside the device, or a combination of valves either passive or active such as solenoid or motorized valves that can control the flow rate in one or both directions. In some embodiments, the inlet is connected to a pump to provide additional gases (not shown in FIG. 1). FIG. 1 shows a single inlet 8. In some embodiments, two or more (e.g., 2, 3, 4, 5, or more) inlets 8 are utilized. In some embodiments, several inlets are connected (e.g., at low pressure regions) along the surface of the device or in parallel or in series or in combination. In some embodiments, the inlet or inlets 8 are tangential to the device, improving the mixing of gases. In some embodiments, the inlet or inlets 8 are tangential to the device, improving the mixing of gases as shown in FIG. 14.

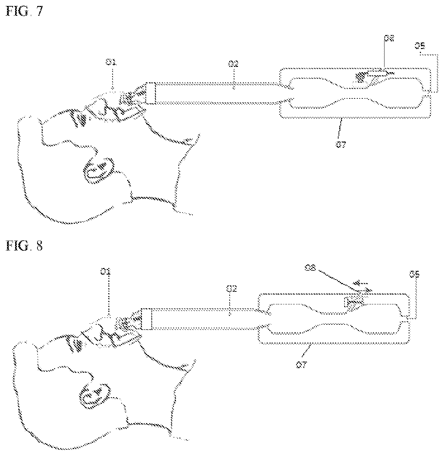

The present disclosure is not limited to a particular location of inlet 8. In FIG. 8, the inlet is shown on the top of mixing chamber 4. However, the inlet 8 may be located in any suitable location of mixing chamber 4.

Still referring to FIG. 1, in the present disclosure primary, secondary, and tertiary chambers are not limited to particular designs of the mixing chamber 4. The primary mixing chamber is proximal to the transport tube, the secondary mixing chamber is proximal to the primary mixing chamber and distal to the tertiary mixing chamber. In some embodiments, the primary mixing chamber 9 is located next to and in operable communication with the transport tube. In some embodiments, the secondary mixing chamber 11 server as a diffuser or conduit to the tertiary mixing chamber 12 that further mixes the gases and stores the gases to be inhaled by the patient in the next respiratory cycle. In some embodiments, the secondary mixing chamber 11 increases the static pressure to reduce the velocity of the excess gases into the atmosphere. In some embodiments, the tertiary mixing chamber 12 stores gases. In some embodiments, the tertiary mixing chamber 12 has a volume of 200 to 2000 ml (e.g., 200, 500, 1000, 1500, or 2000 ml).

Still referring to FIG. 1, the outlet/inlet 5 is where gases return to the atmosphere or enter the device from the atmosphere. In some embodiments, the outlet/inlet 5 comprises a passive or active valve in order to increase the pressure inside the device. In some embodiments, when the valve is closed, all gas intake and outflow flow through port 8.

Still referring to FIG. 1, in some embodiments, device comprises a case 7 to hold the structure in place and to protect the internal structure. In some embodiments, the case 7 is solid, malleable, closed or with apertures. The present disclosure is not limited to particular materials for the case 7. Examples include, but are not limited to, metal, alloys, polymers or wood.

Still referring to FIG. 1, the device comprises a nozzle 10 located at the distal end of the transport tube 2. Nozzle 10 serves to accelerate the exhaled air to create a negative pressure in the chamber, allowing the atmospheric air or other gas or gases to enter the mixing chamber 4. The nozzle diameter can be fixed or variable to control the velocity of the exhaled air and hence the negative pressure and flow rate through transport tube 2. The nozzle 10 shown in FIG. 1 has a converging tip to accelerate the flow and hence increase the negative pressure. However, the present disclosure is not limited to a nozzle 10 with a converging tip. In some embodiments, a nozzle 10 with a constant cross-section is used (e.g., a nozzle with a diameter that is smaller than the mixing chamber 4 diameter). In some embodiments, a converging-diverging nozzle 10 is used. In some embodiments, the nozzle 10 has a lower diameter than the mixing chamber 4 or primary mixing chamber 9. If the nozzle 10 is completely flush with the mixing chamber 4, less mixing occurs with the atmospheric air. If the nozzle 10 is moved away from the entrance to the mixing chamber 4, there is no mixing with gases stored in the mixing chamber 4. Between the two extreme positions, more or less mixing occurs. Although only one nozzle is shown in FIG. 1, in some embodiments, 2 or more (e.g., 2, 3, 4, 5 or more) nozzles are used. Although the inlet 8 is shown in FIG. 1 is radial, a tangential inlet is also suitable, which further improve the mixing of gases reducing the need of longer length to properly mix the gases as shown in FIG. 10.

Figures 2-11 and 13-14 show additional designs and embodiments of the disclosed devices.

Now referring to FIG. 2, shown is a device where air enters the device on the side of transport tube 2 through the sides of the transport tube 2 where it enters the mixing chamber 4 as shown by the arrows. In both FIG. 1 and 2, the amount of atmospheric air that is mixed is controlled by the relative position of the transport tube 2 and mixing chamber 4. When the transport tube 2 is completely flush with the mixing chamber 4, mixing with atmospheric air occurs only at outlet/inlet 5. Thus, the amount of CO₂ in the mixing is maximum.

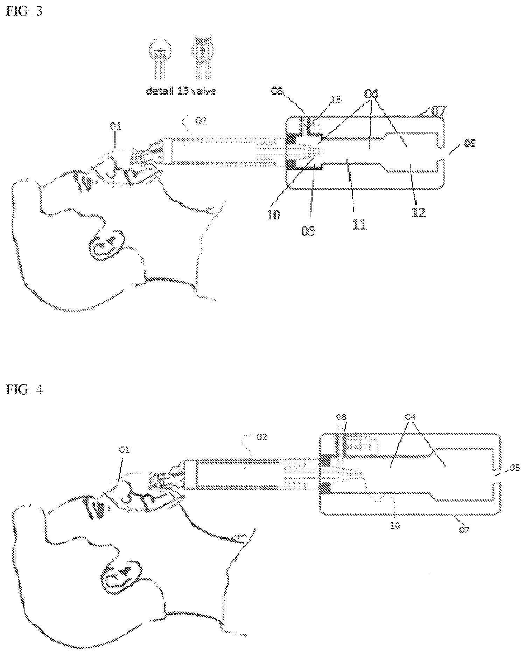

Now referring to FIG. 3, shown is a device that comprises a butterfly valve (or other valve) 13 in the gas inlet 8. In some embodiments, the valve (e.g., butterfly valve) is used in combination with the transport tube adjustment component 3 and/or other components described herein.

Now referring to FIG. 4, shown is a device where the flow of air through gas inlet 8 is controlled by constricting the gas inlet 8 with a screw (shown in FIG. 4) or other component that is able to constrict the gas inlet 8.

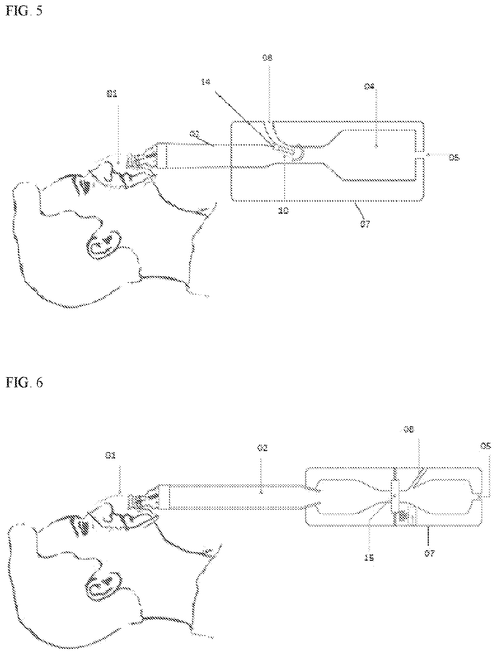

Now referring to FIG. 5, a device is shown where nozzle 10 is generated by an actuator 14. When the actuator 14 rotates counter clockwise it can completely shut the passage through gas inlet 8 so there is no mixing air will occur through outlet/inlet 5. Thus, maximum gas modulation treatment occurs. When the actuator 14 moves clockwise, it becomes a nozzle 10 with variable cross-sectional area depending on the angle or the rotation.

Now referring to FIG. 6, shown is a device where the flow of air is controlled by constricting a region of mixing chamber 4. In FIG. 6, a point of constriction 15 is shown in the center (e.g., symmetrical venturi) of mixing chamber 4. However, the constriction can be placed in any suitable location. When the chamber is constrained in location 15 (e.g., reduced cross-section area) the air velocity increases and hence a negative pressure occurs. The constraint can be done in any way to change the cross-section area including, but not limited to, a butterfly valve, a diaphragm, guillotine valve, pinch valve etc. The negative pressure and hence the secondary flow is controlled by varying cross-section area of the constriction.

Now referring to FIGS. 7 and 8, shown are embodiments where the air control is based on constraint of the gas inlet 8. In the embodiments, shown in FIG. 7, a physical constraint of the gas inlet 8 is used to control flow through the gas inlet 8. However, the present disclosure is not limited to a constraint. Other control component may be used (e.g., a screw as shown in FIG. 4 or a valve as shown in FIG. 8). In the embodiment shown in FIG. 7, the gas inlet 8 is placed in distal portion of a symmetrical venturi mixing chamber. However, the gas inlet 8 can be placed in any suitable location of the reduced cross-sectional area.

Now referring to FIG. 9, shown is a device comprising a blower component 16. The negative pressure and hence the mixing is controlled by an external blower, fan or pump. The negative pressure can be controlled not only by the options described in FIGS. 1-8, but also by the velocity of the blower. The blower can blow the atmospheric air from left to right or from right to left. However, it is more advantageous if it is from left to right since the exhaled air does not get in contact with the blower, hence does not require a filter or constant cleaning of the blower. The present disclosure is not limited to a single blower. In some embodiments, 2 or more blowers are utilized in series or in parallel. The blower can be of any type including, but not limited to, positive displacement, helical screw, centrifugal, regenerative, syringe pump, diaphragm pump, piezoelectric or any other means for displacing air or gases. Commercially available blowers include, but are not limited to, those available from Master Flex (Cole-Parmer, Vernon Hills, IL).

Now referring to Fig. 10, shown is an embodiment where a secondary flow inlet 30 is provided. In the embodiment shown in Fig. 10, the secondary flow inlet 30 enters tangentially to the mixing chamber 31. In some embodiments, this improves the mixing of expired air and atmospheric air.

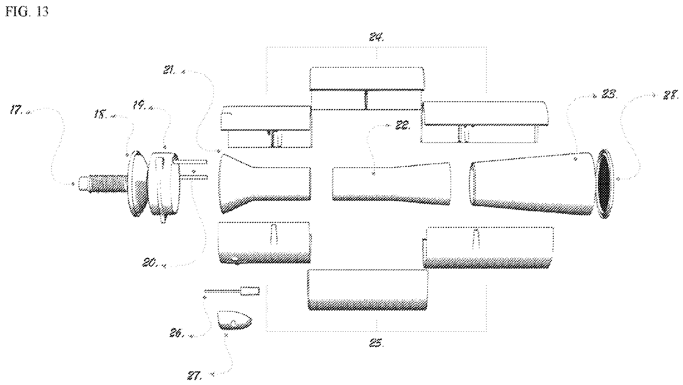

Now referring to FIG. 13, shown is a CAD design of an exemplary device. The inlet 17 guides the respiration flow into the device. The valve 19 (including components 18 and 20) moves right/left to allow more or less mixing with the atmospheric that enters the device. The valve 19 moves according to the movement of the screw motor 26 protected by the cap 27. The protection caps 24 and 25 protect the main body 21, 22 and 23 and the outlet is covered by a filter 28. All parts can be built in one piece but are divided in FIG. 13 to show an exemplary injection molding or 3D print plan.

Now referring to FIG. 14, shows in a physical realization of the CAD design of FIG. 13 plus the mask and hose.

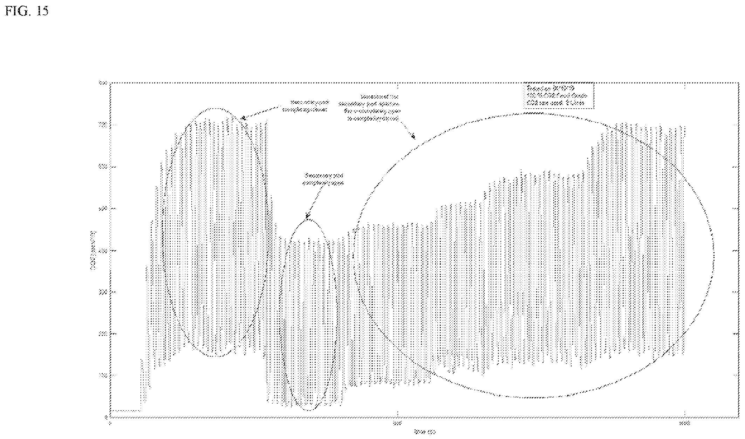

Now referring to FIG. 15, shown are experimental results obtained using the device shown in FIG. 14. Number 1 shows the concentration of CO₂ inside the mixing chamber when the valve is completely closed. Thus, the secondary flow is minimum. Number 2 shows the concentration of CO₂ when the valve is completely open. Hence, the secondary flow is maximum. Number 3 shows the variation of the concentration of CO₂ according the aperture of the valve, from maximum aperture to minimum aperture. In the experiment, a metabolic simulator with mass flow controller (Vacumed, CA) was used. A CO₂ (100% concentration) tank was connected to the simulator at a mass flow that creates a maximum concentration of 7000 ppm of CO₂ when the valve at the secondary flow is completely shut. The simulator was set to a tidal volume of 0.5 Liter/min and respiration rate of 15 respirations/minute, which are typical of a normal adult at rest state.

The present disclosure is not limited to particular sizes of devices. In some embodiments, the effective cross-section area of the device is larger than the cross-section area of a cylinder of 10 mm in diameter in order to avoid extra resistance to normal respiration. However, any specific cross-section area can be used given another path for the flow of gases to occur. For example, the mixing chamber can have a diameter of 0 mm if the transport tube and inlet have effective diameters in combination of at least 15 mm. In this case, all the flow occurs from the inlet and transport tube with minimal addition of excess CO₂ or other gas/gases.

Further provided herein are systems comprising the devices described herein, a CO₂ sensor, and a processor and algorithm that controls CO₂ delivery by the device based on feedback from the CO₂ sensor and/or the patient. In some embodiments, systems include a controller (e.g., controlled by the processor) that controls function of the device in order to modulate CO₂ levels and/or airflow through the device.

The present disclosure is not limited to particular CO₂ sensors. In some embodiments, commercially available CO₂ sensors are utilized (e.g., available from Kele, Memphis, TN or any number of other commercial suppliers). In some embodiments, the CO₂ sensor is located in a suitable location or locations of the device in order measure the level of CO₂ in the mixing chamber (e.g., including but not limited to, internal to the mixing chamber or external with access via an inlet or valve).

In some embodiments, systems include components to detect apnea in a patient. The apneic event may be an apnea or a hypopnea, or the apneic event may be the absence of normal respiration (e.g., the temporary cessation of breathing) or a hypopnea (e.g., abnormally slow or shallow breathing). For example, one or more sensors are provided in the airstream that measure the flow rate of each breath of the wearer and may sense the slowing or cessation of breathing or a reduction in airflow. The sensor may also be a pulse oximeter, a thermal sensor, an optical sensor, or the like, or combinations of the foregoing, as well as combinations of any of the sensors described herein. Accordingly, apneas may be detected, for example by pulse oximetry, or a thermal flow sensor (such as hot wire anemometer), or an optical sensor (such as that detects movement of a drag sensor), or a flow sensor (such as a pneumotachometer).

In some embodiments, when apnea is detected, the controller controls the levels of CO₂ or the negative pressure in the device (e.g., by adjusting one or more valves, blowers, etc.) in order to treat the apnea. In some embodiments, the sensor is configured to take readings at regular intervals (e.g., every microsecond, millisecond, second, minute, or longer intervals). The algorithm and processor then determine if apnea or hypoapnea is present and directs the controller to adjust the device accordingly.

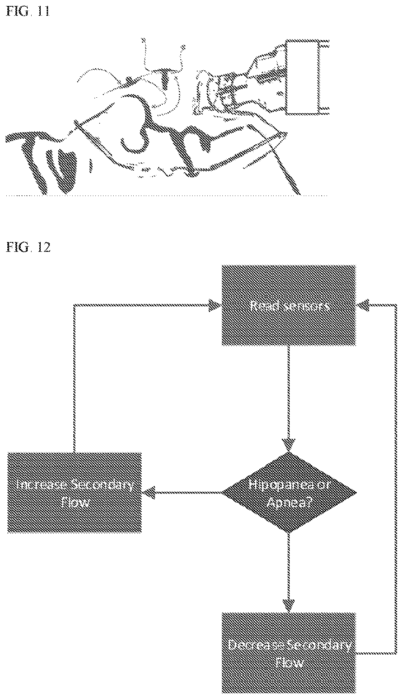

In some embodiments, the controller further controls valves present in a mask as shown in FIG. 11. For example, in some embodiments, when the sensor detects normal respiration, the controller closes all the inlets/outlets of the valve, which decreases the differential pressure inside the mask during inspiration and increases the differential pressure during the respiration phase. In such embodiments, two one-way valves (as shown in FIG. 11) or a two-way valve both with preset open and close pressure will open, allowing the patient to breathe through the mask as shown in FIG. 11.

As described herein, the present disclosure provides methods of treating apnea using the devices and systems described herein. The devices and systems are suitable for treatment of any type of apnea (e.g., obstructive, central or mixed sleep apnea).
Although the disclosure has been described in connection with specific embodiments, it should be understood that the disclosure as claimed should not be unduly limited to such specific embodiments. Indeed, various modifications and variations of the described compositions and methods of the disclosure will be apparent to those of ordinary skill in the art and are within the scope of the following claims.

## Claims

1. A negative air pressure breathing device comprising a mixing chamber (4, 31) configured to move air via a venturi effect, the breathing device comprising: a transport tube (2) configured to transport air, wherein said transport tube (2) further comprises a nozzle (10)
located at a distal end of the transport tube (2), in operable communication with said mixing chamber (4, 31), the nozzle (10) being configured to accelerate exhaled air to create a negative pressure in the chamber, allowing the atmospheric air or other gas or gases to enter the mixing chamber; said at least one mixing chamber comprising a primary (9), a secondary (11), and a tertiary (12) mixing chamber, wherein said primary mixing chamber (9) is proximal to said transport tube (2), said secondary mixing chamber (11) is proximal to said primary mixing chamber (9) and distal to said tertiary mixing chamber (12), wherein said mixing chamber (4, 31) comprises a constrictable region (11), wherein a constriction (15) is configured to generate a negative pressure via the venturi effect; at least one gas inlet (8, 30) in operable communication with said mixing chamber (4, 31); and, in addition to said gas inlet (8, 30) at least one inlet/outlet (5) in operable communication with said mixing chamber (4, 31).

2. The device of claim 1, wherein said device further comprises a face mask (1) in operable communication with said transport tube (2), preferably wherein said face mask (1) comprises one or more valves.

3. The device of any one of claims 1 to 2, wherein said transport tube (2) further comprises an adjustment component (3) configured to move said transport tube (2) in closer or further proximity to said mixing chamber (4, 31), preferably wherein said adjustment component (3) is an infinity screw or a motor.

4. The device of any one of claims 1 to 3, wherein:
- said inlet further comprises a valve configured to control the cross-sectional area of said inlet, preferably wherein said valve
is a two-way valve, preferably a butterfly valve, or wherein said valve is a one-way valve, preferably an umbrella valve; or
wherein said valve is configured to move along an axis of the inlet, preferably wherein said valve is moved by a screw motor.

5. The device of any one of claims 1 to 4, wherein said inlet is located on the top of said mixing chamber (4, 31) and/or on the side of said transport tube (2); and/or wherein said inlet comprises an actuator that controls movement of air through said actuator.

6. The device of any one of claims 1 to 5, wherein said device comprises two or more inlets and/or outlets, preferably wherein said outlet is covered by a filter (28).

7. The device of any one of claims 1 to 6, wherein said device further comprises a secondary flow inlet, preferably wherein said secondary flow inlet enters said device tangentially to said mixing chamber (4, 31).

8. The device of any one of claims 1 to 7, wherein a constriction (15) generates a negative pressure via the venturi effect, preferably wherein said constriction (15) is controlled by a valve or diaphragm.

9. The device of any one of claims 1 to 8, wherein said device further comprises an external source of gas in operable communication with said device.

10. The device of any one of claims 1 to 9, wherein said device provides intermittent, periodic, or continuous negative air pressure.

11. The device of any one of claims 1 to 10, wherein said device traps CO₂ in said mixing chamber (4, 31).

12. A system, comprising:
a) the device of any one of claims 1 to 11;
b) at least one sensor selected from the group consisting of a CO₂ sensor, a pressure sensor, a temperature sensor, and an air flow sensor; and
c) a processor configured to use an algorithm to control CO₂ levels or airflow provided by said device;
preferably wherein said system further comprises a controller configured to control said device and modulate CO₂ levels or airflow.

13. The system of claim 12, wherein said processor controls CO₂ levels or airflow by controlling flow into said device through said inlet.

14. Device according to any one of claims 1 - 11 or system according to any one of claims 12 to 13 for use in the treatment of sleep apnea.

15. Device or system according to claim 14, for use in the treatment of obstructive, central or mixed sleep apnea.

## Patentansprüche

1. Unterdruckatmungsvorrrichtung, umfassend eine Mischkammer (4, 31), die dazu ausgelegt ist, Luft über einen Venturi-Effekt zu bewegen, wobei die Atmungsvorrichtung umfasst: eine Transportröhre (2), die dazu ausgelegt ist, Luft zu transportieren, wobei die Transportröhre (2) ferner eine Düse (10) umfasst, die sich an einem distalen Ende der Transportröhre (2) in funktioneller Verbindung mit der Mischkammer (4, 31) befindet, wobei die Düse (10) dazu ausgelegt ist, Ausatemluft zu beschleunigen, um einen Unterdruck in der Kammer zu erzeugen, was erlaubt, dass atmosphärische Luft oder anderes Gas oder andere Gase in die Mischkammer gelangen; wobei die mindestens eine Mischkammer eine primäre (9), eine sekundäre (11) und eine tertiäre (12) Mischkammer umfasst, wobei die primäre Mischkammer (9) proximal zu der Transportröhre (2) ist, die sekundäre Mischkammer (11) proximal zu der primären Mischkammer (9) und distal zu der tertiären Mischkammer (12) ist, wobei die Mischkammer (4, 31) einen verengbaren Bereich (11) umfasst, wobei eine Verengung (15) dazu ausgelegt ist, über den Venturi-Effekt einen Unterdruck zu erzeugen; mindestens einen Gaseinlass (8, 30) in funktioneller Verbindung mit der Mischkammer (4, 31); und zusätzlich zu dem Gaseinlass (8, 30) mindestens einen Einlass/Auslass (5) in funktioneller Verbindung mit der Mischkammer (4, 31).

2. Vorrichtung nach Anspruch 1, wobei die Vorrichtung ferner eine Gesichtsmaske (1) in funktioneller Verbindung mit der Transportröhre (2) umfasst, wobei vorzugsweise die Gesichtsmaske (1) ein oder mehrere Ventile umfasst.

3. Vorrichtung nach einem der Ansprüche 1 bis 2, wobei die Transportröhre (2) ferner eine Verstellkomponente (3) umfasst, die dazu ausgelegt ist, die Transportröhre (2) in größere oder weitere Nähe zu der Mischkammer (4, 31) zu bewegen, wobei vorzugsweise die Verstellkomponente (3) eine Infinity-Schraube oder ein Motor ist.

4. Vorrichtung nach einem der Ansprüche 1 bis 3, wobei:
- der Einlass ferner ein Ventil umfasst, das dazu ausgelegt ist, die Querschnittsfläche des Einlasses zu steuern, wobei vorzugsweise das Ventil ein Zweiwegeventil, vorzugsweise ein Schmetterlingsventil, ist oder wobei das Ventil ein Einwegventil, vorzugsweise ein Schirmventil, ist; oder
wobei das Ventil dazu ausgelegt ist, sich entlang einer Achse des Einlasses zu bewegen, wobei vorzugsweise das Ventil von einem Schneckenmotor bewegt wird.

5. Vorrichtung nach einem der Ansprüche 1 bis 4, wobei sich der Einlass oben an der Mischkammer (4, 31) und/oder an der Seite der Transportröhre (2) befindet; und/oder wobei der Einlass eine Betätigungseinrichtung umfasst, die die Bewegung von Luft durch die Betätigungseinrichtung steuert.

6. Vorrichtung nach einem der Ansprüche 1 bis 5, wobei die Vorrichtung zwei oder mehr Einlässe und/oder Auslässe umfasst, wobei vorzugsweise der Auslass von einem Filter (28) abgedeckt ist.

7. Vorrichtung nach einem der Ansprüche 1 bis 6, wobei die Vorrichtung ferner einen sekundären Strömungseinlass umfasst, wobei vorzugsweise der sekundäre Strömungseinlass tangential zur Mischkammer (4, 31) in die Vorrichtung gelangt.

8. Vorrichtung nach einem der Ansprüche 1 bis 7, wobei eine Verengung (15) über den Venturi-Effekt einen Unterdruck erzeugt, wobei vorzugsweise die Verengung (15) von einem Ventil oder einer Membran gesteuert wird.

9. Vorrichtung nach einem der Ansprüche 1 bis 8, wobei die Vorrichtung ferner eine externe Gasquelle in funktioneller Verbindung mit der Vorrichtung umfasst.

10. Vorrichtung nach einem der Ansprüche 1 bis 9, wobei die Vorrichtung intermittierenden, periodischen oder kontinuierlichen Luftunterdruck bereitstellt.

11. Vorrichtung nach einem der Ansprüche 1 bis 10, wobei die Vorrichtung in der Mischkammer (4, 31) CO₂ speichert.

12. System, umfassend:
a) die Vorrichtung nach einem der Ansprüche 1 bis 11;
b) mindestens einen Sensor, der aus der Gruppe bestehend aus einem CO₂-Sensor, einem Drucksensor, einem Temperatursensor und einem Luftstromsensor ausgewählt ist; und
c) einen Prozessor, der dazu ausgelegt ist, einen Algorithmus zu verwenden, um den CO₂-Gehalt oder den Luftstrom zu steuern, der von der Vorrichtung bereitgestellt wird;
wobei vorzugsweise das System ferner eine Steuerung umfasst, die dazu ausgelegt ist, die Vorrichtung zu steuern und den CO₂-Gehalt oder den Luftstrom zu modulieren.

13. System nach Anspruch 12, wobei der Prozessor den CO₂-Gehalt oder Luftstrom steuert, indem er die Strömung durch den Einlass in die Vorrichtung steuert.

14. Vorrichtung nach einem der Ansprüche 1-11 oder System nach einem der Ansprüche 12 bis 13 zur Verwendung in der Behandlung von Schlafapnoe.

15. Vorrichtung oder System nach Anspruch 14 zur Verwendung in der Behandlung von obstruktiver, zentraler oder gemischter Schlafapnoe.

## Revendications

1. Dispositif respiratoire à pression d'air négative comprenant une chambre de mélange (4, 31) configurée pour déplacer l'air par un effet venturi, le dispositif respiratoire comprenant : un tube de transport (2) configuré pour transporter de l'air, dans lequel ledit tube de transport (2) comprend en outre une buse (10) située à une extrémité distale du tube de transport (2), en communication fonctionnelle avec ladite chambre de mélange (4, 31), la buse (10) étant configurée pour accélérer l'air expiré pour créer une pression négative dans la chambre, permettant à l'air atmosphérique ou à un autre/d'autres gaz d'entrer dans la chambre de mélange ; ladite au moins une chambre de mélange comprenant une chambre de mélange primaire (9), secondaire (11) et tertiaire (12), dans lequel ladite chambre de mélange primaire (9) est proximale audit tube de transport (2), ladite chambre de mélange secondaire (11) est proximale à ladite chambre de mélange primaire (9) et distale par rapport à ladite chambre de mélange tertiaire (12), dans lequel ladite chambre de mélange (4, 31) comprend une région pouvant se rétrécir (11), dans lequel un rétrécissement (15) est configuré pour générer une pression négative par l'effet venturi ; au moins une entrée de gaz (8, 30) en communication fonctionnelle avec ladite chambre de mélange (4, 31) ; et, en plus de ladite entrée de gaz (8, 30), au moins une entrée/sortie (5) en communication fonctionnelle avec ladite chambre de mélange (4, 31).

2. Dispositif de la revendication 1, dans lequel ledit dispositif comprend en outre un masque facial (1) en communication fonctionnelle avec ledit tube de transport (2), de préférence dans lequel ledit masque facial (1) comprend une ou plusieurs vannes.

3. Dispositif de l'une quelconque des revendications 1 et 2, dans lequel ledit tube de transport (2) comprend en outre un composant de réglage (3) configuré pour déplacer ledit tube de transport (2) plus près ou plus loin de ladite chambre de mélange (4, 31), de préférence dans lequel ledit composant de réglage (3) est une vis sans fin ou un moteur.

4. Dispositif de l'une quelconque des revendications 1 à 3, dans lequel :
- ladite entrée comprend en outre une vanne configurée pour commander la surface de section transversale de ladite entrée, de préférence dans lequel ladite vanne
est une vanne à deux voies, de préférence une vanne à papillon, ou dans lequel ladite vanne est une vanne unidirectionnelle, de préférence une vanne parapluie ; ou
dans lequel ladite vanne est configurée pour se déplacer le long d'un axe de l'entrée, de préférence dans lequel ladite vanne est déplacée par un moteur à vis.

5. Dispositif de l'une quelconque des revendications 1 à 4, dans lequel ladite entrée est située au sommet de ladite chambre de mélange (4, 31) et/ou sur le côté dudit tube de transport (2) ; et/ou dans lequel ladite entrée comprend un actionneur qui commande le mouvement de l'air à travers ledit actionneur.

6. Dispositif de l'une quelconque des revendications 1 à 5, dans lequel ledit dispositif comprend deux entrées et/ou sorties ou plus, de préférence dans lequel ladite sortie est recouverte d'un filtre (28).

7. Dispositif de l'une quelconque des revendications 1 à 6, dans lequel ledit dispositif comprend en outre une entrée de débit secondaire, de préférence dans lequel ladite entrée de débit secondaire entre dans ledit dispositif tangentiellement à ladite chambre de mélange (4, 31).

8. Dispositif de l'une quelconque des revendications 1 à 7, dans lequel un rétrécissement (15) génère une pression négative par l'effet venturi, de préférence dans lequel ledit rétrécissement (15) est commandé par une vanne ou un diaphragme.

9. Dispositif de l'une quelconque des revendications 1 à 8, dans lequel ledit dispositif comprend en outre une source externe de gaz en communication fonctionnelle avec ledit dispositif.

10. Dispositif de l'une quelconque des revendications 1 à 9, dans lequel ledit dispositif fournit une pression d'air négative intermittente, périodique ou continue.

11. Dispositif de l'une quelconque des revendications 1 à 10, dans lequel ledit dispositif piège le CO₂ dans ladite chambre de mélange (4, 31).

12. Système, comprenant :
a) le dispositif de l'une quelconque des revendications 1 à 11 ;
b) au moins un capteur choisi dans le groupe constitué d'un capteur de CO₂, d'un capteur de pression, d'un capteur de température et d'un capteur de débit d'air, et
c) un processeur configuré pour utiliser un algorithme pour commander les niveaux de CO₂ ou le débit d'air fourni par ledit dispositif ;
de préférence, dans lequel ledit système comprend en outre un dispositif de commande configuré pour commander ledit dispositif et moduler les niveaux de CO₂ ou le débit d'air.

13. Système de la revendication 12, dans lequel ledit processeur commande les niveaux de CO₂ ou le débit d'air en commandant le débit dans ledit dispositif à travers ladite entrée.

14. Dispositif selon l'une quelconque des revendications 1 à 11 ou système selon l'une quelconque des revendications 12 et 13 pour une utilisation dans le traitement de l'apnée du sommeil.

15. Dispositif ou système selon la revendication 14, pour une utilisation dans le traitement de l'apnée obstructive, centrale ou mixte du sommeil.
